(19) Europäisches Patentamt
European Patent Office
Office européen des brevets

(11) Publication number: **0 064 487**
**A1**

(12) **EUROPEAN PATENT APPLICATION**

(21) Application number: 82850093.4

(22) Date of filing: 30.04.82

(51) Int. Cl.³: C 07 C 93/06
A 61 K 31/13

(30) Priority: 06.05.81 SE 8102823

(43) Date of publication of application:
10.11.82 Bulletin 82/45

(84) Designated Contracting States:
AT BE CH DE FR GB IT LI LU NL SE

(71) Applicant: Aktiebolaget Hässle
Kärragatan 5
S-431 83 Mölndal(SE)

(72) Inventor: Carlsson, Enar Ingemar
Ingegärdsvägen 2 C
S-421 68 Västra Frölunda(SE)

(72) Inventor: Gustafsson, Bill Benjamin Rudolf
Krokdalsvägen 106
S-517 00 Bollebygd(SE)

(72) Inventor: Källsson, Britt Inger Monica
Krokslätts parkgata 53 A
S-431 38 Mölndal(SE)

(72) Inventor: Mattsson, Annie Hillevi
Pilegården 9 C
S-436 00 Askim(SE)

(74) Representative: Wurm, Bengt Runio et al,
Patent and Trade Mark Department Ab Astra
S-151 85 Södertälje(SE)

(54) **New substituted 3-(parasubstituted)phenoxy-1-alkylamino-propanol-2-s, as well as process for their preparation, pharmaceutical preparations containing same, and method for treating cardiac failure.**

(57) Compounds of the formula I

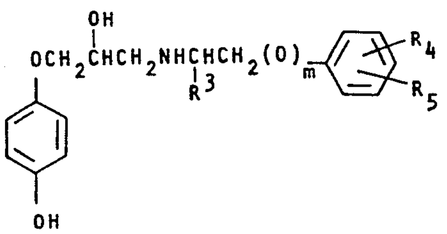

(1)

wherein $R^3$ is selected from the group consisting of hydrogen or methyl, m is an integer 0 or 1, whereby when m is 0, $R^4$ and $R^5$ are the same and are selected from the group consisting of methoxy, fluoro, bromo, and chloro in 2- and 6-position, whereby when m is 1, $R^4$ is hydrogen and $R^5$ is selected from the group consisting of hydrogen, methoxy, ethoxy, acetyl, methyl, hydroxymethyl, cyano, chloro, fluoro, bromo, or hydroxy, or a pharmaceutically acceptable salt thereof, as well as their preparation, pharmaceutical preparations containing same, and method of treating cardiac failure.

The compounds which possess beta-adrenoceptor stimulating activity are intended for treatment of cardiac disease whereby the compounds possess a pronounced inotropic effect.

EP 0 064 487 A1

# New substituted 3-(parasubstituted)phenoxy-1-alkylamino-propanol-2-s, as well as process for their preparation, pharmaceutical preparations containing same, and method for treating cardiac failure

## DESCRIPTION

### TECHNICAL FIELD

The present invention relates to new compounds with β-adrenoceptor stimulating properties, process for their preparation, pharmaceutical preparations containing said compounds and methods for treatment of cardiac failure.

The object of the invention is to obtain new drugs with a positive inotropic action on the heart which can be used for treatment of cardiac failure.

### BACKGROUND OF THE INVENTION

Many heart diseases create such extensive damage to the muscle of the myocardium that a heart insufficiency occurs. A fundamental component in the therapy of heart insufficiency is an agent which has a positive inotropic action on the heart, i.e. one which increases the contractile force of the heart and/or which has a positive chronotropic action on the heart i.e. one which increases the beating frequency. Presently the most commonly used agents are the glycosides of digitalis which have inotropic action. However, the digitalis preparations show evident drawbacks from a therapeutic point of view. They have a low therapeutic index and cause cardiac arrhythmias at dosages insignificantly higher than those needed for a positive inotropic effect. Thus, there is an evident therapeutic need of inotropic agents which can replace or complement the digitalis preparations.

Normally the pumping activity of the heart is stimulated via the sympathetic nervous system by release of noradrenaline from the cardiac sympathetic nerves and by adrenaline from the adrenal medulla. The action on the heart is mediated via $\beta_1$-adrenoceptors. However, both noradrenaline and adrenaline as well as the synthetic β-adrenoceptor agonist isoprenaline have other effects on the circulatory system. Noradrenaline and adrenaline are strong constrictors of blood vessels while isoprenaline is a strong dilator. Furthermore, the three compounds are rapidly eliminated by metabolism and therefore very shortacting.

The compounds of the present invention have stimulating effects on the heart related to those of the endogenous catecholamines and isoprenaline but are devoid of their vascular effects. Furthermore, the present compounds are longacting.

DISCLOSURE OF THE INVENTION

It has been found that the compounds of the general formula

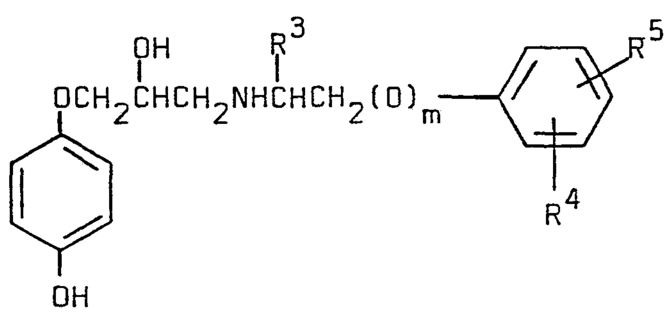

I

wherein $R^3$ is selected from the group consisting of hydrogen or methyl, m is an integer 0 or 1, whereby when m is 0, $R^4$ and $R^5$ are the same and selected from the group consisting of methoxy, fluoro, bromo, and chloro in 2- and 6-position, whereby when m is 1, $R^4$ is hydrogen and $R^5$ is selected from the group consisting of hydrogen, methoxy, ethoxy, acetyl, methyl, hydroxymethyl, cyano, chloro, fluoro, bromo, hydroxy, and pharmaceutically acceptable acid addition salts thereof

have valuable pharmacological properties and may fulfil the above-mentioned objects.

The substances are intended to be administered orally or parenterally for acute and chronic treatment of cardiac failure, specifically ventricular myocardial failure (disease conditions with a diminution in ventricular contractility below that of a normal heart such that the capacity of the myocardium, at any given fiber length, to develop tension or to shorten against a load is so impaired as to compromise the circulation). Accordingly, the substances are intended to improve symptoms and signs of cardiac failure such as dyspnea, cyanosis, pulmonary edema, increased venous pressure, liver enlargement, and peripheral edema.

The substances may be used alone and in combination with other therapeutic measures, such as administration of digitalis and diuretic drugs. Also, the substances may be used in combination with other measures in treating cardiogenic shock, the condition associated with reduced arterial blood pressure which often complicates myocardial infarction. Another use for the substances is in treatment of bradycardia, that is, conditions with slow heart rhythm where the weak positive chronotropic effect in combination with the positive inotropic effect of the substances can be expected to be of therapeutic value.

Compounds of the present invention of special interest are:

1) 1-[2-(2,6-dimethoxyphenyl)-1-methylethylamino]-3-(4-hydroxyphenoxy)propanol-2
2) 3-(4-hydroxyphenoxy)-1-[2-(4-hydroxyphenoxy)-1-methylethylamino]propanol-2
3) 1-[2-(2,6-dichlorophenyl)-1-methylethylamino]-3-(4-hydroxyphenoxy)propanol-2

4) 3-(4-hydroxyphenoxy)-1-[2-(3-hydroxyphenoxy)-1-methylethylamino]propanol-2

5) 3-(4-hydroxyphenoxy)-1-[2-(3-methoxyphenoxy)-1-methylethylamino]propanol-2

6) 1-[2-(3-chlorophenoxy)-1-methylethylamino]-3-(4-hydroxyphenoxy)propanol-2

7) 1-[2-(3-hydroxyphenoxy)ethylamino]-3-(4-hydroxyphenoxy)-propanol-2

8) 1-(2-phenoxy-1-methylethylamino)-3-(4-hydroxyphenoxy)-propanol-2

9) 1-(2-phenoxyethylamino)-3-(4-hydroxyphenoxy)propanol-2

10) 1-[2-(2-hydroxyphenoxy)ethylamino]-3-(4-hydroxyphenoxy)-propanol-2

11) 1-[2-(2-chlorophenoxy)ethylamino]-3-(4-hydroxyphenoxy)-propanol-2

12) 3-(4-hydroxyphenoxy)-1-[2-(2-methoxyphenoxy)ethylamino]-propanol-2.

According to one preferred embodiment the invention is related to compounds of formula I, wherein m is 0 and $R^4$ and $R^5$ are the same and selected from methoxy, fluoro, bromo and chloro in the 2- and 6-position.

According to another preferred embodiment the invention is related to compounds of formula I, wherein m is 1, $R^4$ is H and $R^5$ is selected from hydrogen, methoxy, chloro, fluoro, bromo and hydroxy. Of those compounds are especially preferred compounds having the formula

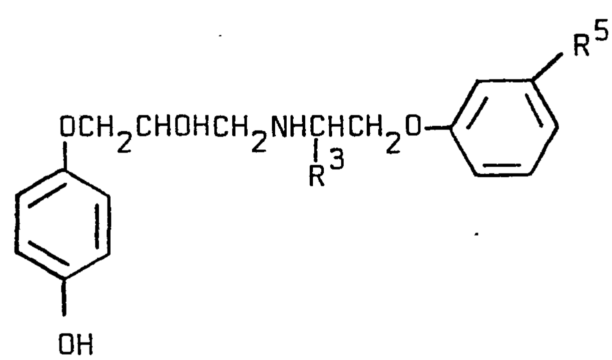

wherein $R^3$ is hydrogen or methyl and $R^5$ is selected from hydrogen, fluoro, chloro, bromo and hydroxy.

Preferred specific compounds are those numbered 1), 3), 4), 6), 7), 8) and 9) above.

Salt forming acids may be used in preparing therapeutically acceptable salts of the compounds. Such acids are: hydrohalogen acids, sulphuric acids, phosphoric acid, nitric acid, perchloric acid, aliphatic, alicyclic, aromatic or heterocyclic carboxy or sulphonic acids, such as formic, acetic, propionic, succinic, glycolic, lactic, malic, tartaric, citric, ascorbic, maleic, hydroxymaleic, or pyruvic acid, phenylacetic, benzoic, p-aminobenzoic, anthranilic, p-hydroxybenzoic, salicyclic or p-aminosalicyclic acid, pamoic acid, methanesulfonic, ethanesulfonic, hydroxyethanesulfonic, ethylenesulfonic, halogenbenzenesulfonic, toluenesulfonic, naphtylsulfonic or sulfanilic acid, methionine, tryptophane, lysine or arginine.

The new compounds are obtained according to methods known <u>per se</u>. Thus, (a) a compound of the formula II

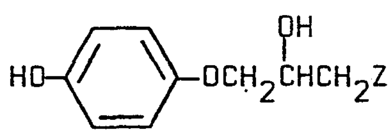

II

wherein Z is OH or an esterified, reactive hydroxy group, or -OH and Z together form an epoxy group, is reacted with an amine of the formula III

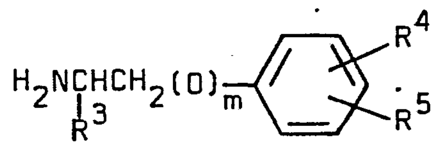

III

wherein $R^3$, $R^4$, $R^5$, and m have the meanings given above.

A reactive, esterified hydroxy group is particularly a hydroxy group esterified with a strong, inorganic or organic acid, preferably a hydrohalogen acid, as hydrochloric acid, hydrobromic acid, or hydroiodic acid, further sulfuric acid or a strong organic sulfonic acid, e.g. benzenesulfonic acid, 4-bromobenzenesulfonic acid, or 4-toluenesulfonic acid. Thus, Z is preferably chloro, bromo or iodo.

This reaction is carried out in a common way. At the use of a reactive ester as a starting material the preparation takes place preferably in the presence of a basic condensating agent and/or with an excess of an amine. Suitable basic condensating agents are e.g. alkalimetal hydroxides as sodium or potassium hydroxide, alkalimetal carbonates as potassium carbonate and alkalimetal alcoholates as sodium methylate, potassium ethylate and potassium tert. butylate.

The reaction is preferably carried out in an alkanol having 1 to 4 carbon atoms by refluxing the reactants in said solvent for a time long enough to give the compound of formula I, generally 1 to 12 hours. However, the reaction can be carried out in the presence of an excess of amine alone. The reaction can also take place in an autoclave.

When Z is a hydroxy group the reaction is performed in the presence of a metal catalyst, preferably Raney nickel.

Further, (b) a compound of formula IV

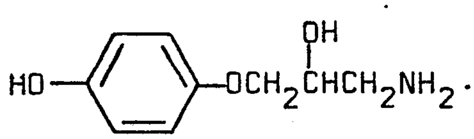

IV

is reacted with a compound of the formula V

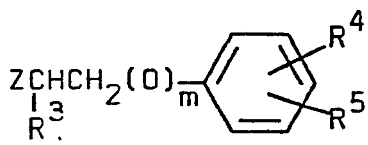

V

or with a compound of formula VI

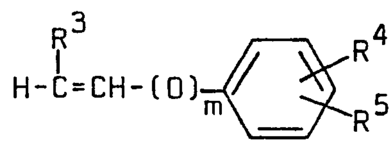

VI

or with a compound of formula VII

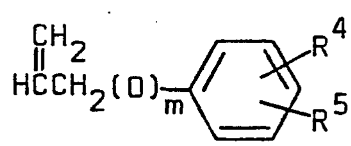

VII

wherein Z, $R^3$, $R^4$, $R^5$, and m have the meanings given above.

This reaction is carried out in a common way, preferably in the presence of a basic condensating agent and/or an excess of an amine, by using a metal catalyst e.g. Raney nickel as a condensating agent (Z = HO-) or by amino-mercuration (reaction between amine and olefin).

Suitable basic condensating agents are e.g. alkaline alcoholates, preferably sodium or potassium alcoholate, or also alkaline carbonates as sodium or potassium carbonate.

Further, (c) a compound of formula VIII

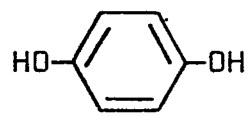

VIII

is reacted with a compound of the formula IX

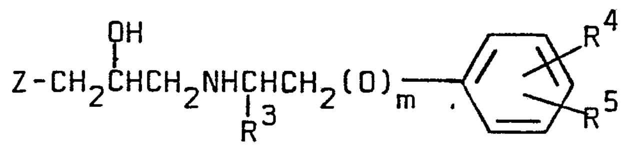

IX

wherein Z, $R^3$, $R^4$, $R^5$, and m have the meanings given above.

Further, (e) a compound of the formula X

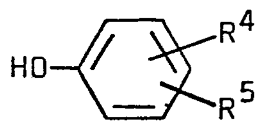

X

wherein $R^4$ and $R^5$ have the meanings given above, is reacted with a compound of the formula XI

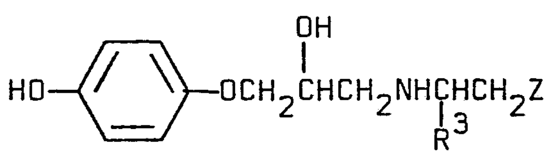

XI

wherein $R^3$ and Z have the meanings given above.

These two latter reactions are carried out in a common way. In those cases where reactive esters are used as starting material, the compound of formula VIII may suitably be used

in the form of its metalphenolate as alkalimetalphenolate, preferably sodiumphenolate, or one works in the presence of an acid binding agent, preferably a condensating agent, which can form a salt of the compound of formula VIII as an alkalimetal alcoholate.

This reaction is preferably carried out in an alkanol having 1 to 3 carbon atoms in an autoclave being heated to 80 to 100°C for 1 to 15 hours.

Further, (d) a compound of formula VIII

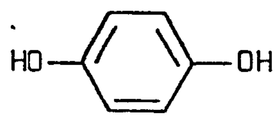

VIII

is reacted with a compound of formula XII

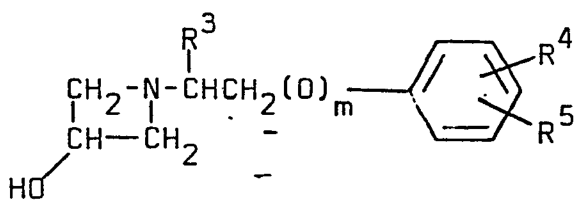

XII

wherein $R^3$, $R^4$, $R^5$, and m have the meanings given above.

This reaction is carried out in a common way. Thus, the reaction is carried out under alkaline conditions in a suitable solvent, as benzylalcohol by boiling the reaction mixture for some hours. Thereby the phenol is primarily converted to its metalphenolate as alkalimetalphenolate before it is added to the acetidinol of formula XII.

Further, (f) one may split off a residue from a compound of formula I above, in which the nitrogen atom of the amino group and/or the hydroxy groups have attached thereto a splitable residue.

Such splitable residues are especially those which are splitable by solvolysis, reduction, pyrolysis or fermentation.

Residues splitable by means of hydrolysis are e.g. an acyl residue, which, when present, also can be functionally varied carboxy groups, e.g. oxycarbonyl residues, as alkoxycarbonyl residues, e.g. tert. butoxycarbonyl residue, or ethoxycarbonyl residue, aralkoxycarbonyl residues as phenylloweralkoxycarbonyl residues, e.g. a carbobenzyloxy residue, halogencarbonyl residue, e.g. a chlorocarbonyl residue, and carbamoyl groups. Further, arylsulphonyl residues as toluenesulphonyl or bromobenzenesulphonyl residues and possibly as halogenated, as fluorinated lower alkanoyl residues as formyl, acetyl or trifluoroacetyl residues or a benzyl residue or cyano groups or silyl residues, as trimethylsilyl residue.

Of the above mentioned residues present at the hydroxy groups, which residues are splitable by hydrolysis, preferably the oxycarbonyl residues and the loweralkanoyl residues or the benzoyl residues are used.

Besides the above mentioned also double-bound residues, which are splitable at the amino group by hydrolysis are used, e.g. alkylidene or benzylidene residue or a phosphorylidene group as a triphenylphosphorylidene group, whereby the nitrogen atom then obtains a positive charge.

Residues splitable at the amino group by hydrolysis are furthermore divalent residues as in occurring cases substituted methylene. As substituents on the methylene residues any organic residue may be used, whereby it does not matter at the hydrolysis which compound is the substituent to the methylene residue. As methylene substituents e.g. aliphatic or aromatic residues as alkyl as mentioned above, aryl e.g. phenyl or pyridyl may be used.

The hydrolysis may be carried out in any common way, suitably in a basic or preferably in an acid medium.

Compounds having residues being splitable by hydrolysis are also the compounds according to formula XIII

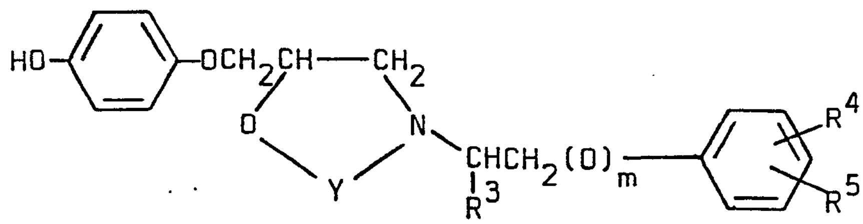

XIII

wherein $R^3$, $R^4$, $R^5$, and m have the same meanings as given above and Y is a carbonyl, thiocarbonyl, or a

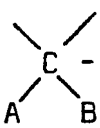 - residue, wherein A and B are each severally hydrogen, alkyl, alkylaryl or aryl.

The hydrolysis is carried out in an analogous way, e.g. in the presence of a hydrolysing agent, e.g. in the presence of an acidic agent as e.g. diluted mineral acids, as sulfuric acid or hydrohalogen acid, or in the presence of basic agents as e.g. alkalimetal hydroxides, as sodium hydroxide. Oxycarbonyl residues, aryl sulfonyl residues and cyano groups may in a suitable way be split off by means of acidic agents as by means of a hydrohalogen acid, suitably hydrobromic acid. Preferably the splitting may take place using diluted hydrobromic acid, possibly in a mixture with acetic acid. Cyano groups are preferably split off by means of hydrobromic acid at an elevated temperature, as in boiling hydrobromic acid, according to the "bromo-cyano method" (v. Braun). Further, e.g. a tert. butoxycarbonyl residue may be split off under anhydrous conditions by means of a treatment with a suitable acid, as trifluoracetic acid.

Acidic agents are preferably used at a hydrolysis of compounds of formula XIII.

Residues splitable by ammonolysis are especially the halogencarbonyl residues, as the chlorocarbonyl residue. The ammonolysis may be carried out in a common way, e.g. by means of an amine containing at least one hydrogen atom bound to the nitrogen atom, as a mono- or diloweralkylamine e.g. methylamine or dimethylamine, or especially ammonia, preferably at an elevated temperature. Instead of ammonia one may use an agent which gives ammonia as hexamethylene-tetraamine.

Residues splitable by means of a reduction are e.g. an α-arylalkyl residue, as a benzyl residue or an α-aralkoxy-carbonyl residue as a benzyloxycarbonyl residue, which in a common way may be split off by means of a hydrogenolysis, especially by catalytically activated hydrogen, as by hydrogen in the presence of hydrogenating catalysts, e.g. Raney-nickel. Further residues splitable by means of hydrogenolysis are 2-halogenalkoxycarbonyl residues as 2,2,2-trichloroethoxycarbonyl residues or 2-iodoethoxy- or 2,2,2-tri-bromoethoxycarbonyl residues, which may be split off in a common way, suitably by means of a metallic reduction (so called nascerating hydrogen). Nascerating hydrogen may be obtained by the influence of metal or metal alloys, as amalgam on compounds which give hydrogen as carboxylic acids, alcohols or water, whereby especially zink or zinkalloys together with acetic acid may be used. Hydrogenolysis of 2-halogenalkoxycarbonyl residues may further take place using chromium or chromium (II) compounds as chromium (II) chloride or chromium (II) acetate.

A residue splitable by reduction may also be an arylsulphonyl group as a toluenesulphonyl group, which in a common way may be split off by reduction using nascerating hydrogen, e.g. by means of an alkalimetal, as lithium or

sodium in liquid ammonia, and suitably may be split off from a nitrogen atom. At the carrying out of the reduction one has to take care that other reducible groups are not influenced.

Residues splitable by means of pyrolysis, especially residues splitable from the nitrogen atom, are in occurring cases substituted suitably unsubstituted carbamoyl groups. Suitable substituents are e.g. loweralkyl or arylloweralkyl as methyl or benzyl or aryl, as phenyl, the pyrolysis is carried out in a common way, whereby one may have to take care of other thermically susceptible groups.

Residues splitable by means of fermentation, especially residues splitable from the nitrogen atom are in occurring cases substituted, however, suitably unsubstituted carbamoyl groups. Suitable substituents are e.g. loweralkyl or arylloweralkyl, as methyl or benzyl, or aryl as phenyl. The fermentation is carried out in a common way, e.g. by means of the enzyme urease or soy bean extract at about 20°C or slightly elevated temperature.

Further, (g) a Schiff's base of formula XIV or XV

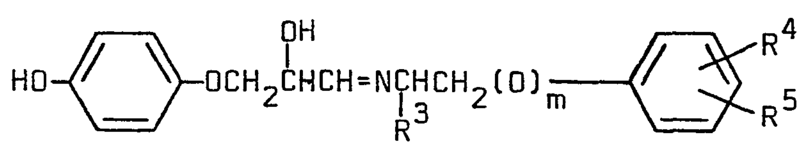

XIV

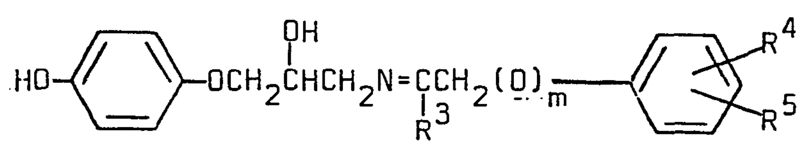

XV

or a cyclic tautomer corresponding to formula XV of formula XVI

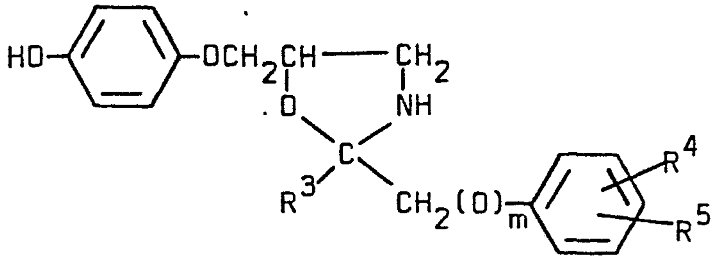

XVI

or a compound corresponding to formula I and having an unsaturation α to the amino nitrogen can be reduced, wherein $R^3$, $R^4$, $R^5$, and m have the same meanings as given above, and whereby the compounds of formula XV and XVI may exist together, too.

This reduction is carried out in a common way, e.g. using a di-lightmetalhydride, as sodium borohydride, lithium aluminium hydride, using a hydride as Boran with formic acid, or by means of a catalytic hydrogenation, as with hydrogen in the presence of Raney-nickel. At the reduction one has to take care that other groups are not affected.

The reduction of the compound of formula XV above can also take place with $CH_3M$, wherein M is a metal e.g. Mg, Li, for $R^3$ being hydrogen in formula XV to produce a compound of formula I.

A compound corresponding to formula I and having an unsaturation α to the amino nitrogen may be a compound of formulae XVII and XVIII which can be reduced in a common way e.g. by means of catalytic hydrogenation or with diboran

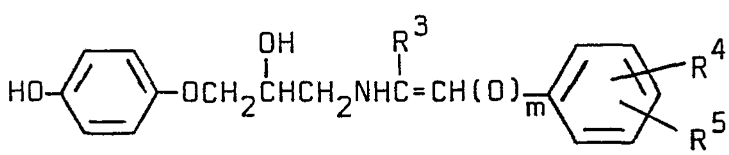

XVII

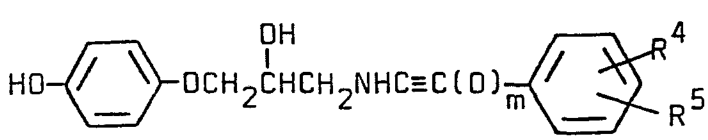

XVIII

Further, (h) the oxo group in the compound of formula XIX

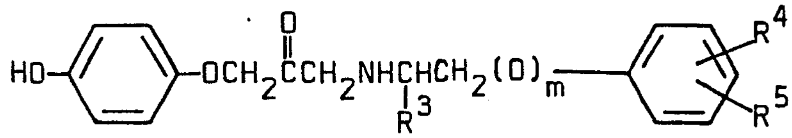

XIX

wherein $R^3$, $R^4$, $R^5$, and m have the same meanings as given above, can be reduced to a hydroxy group. This reduction is carried out in a common way, especially using a di-lightmetalhydride, as mentioned above, or according to the "Meerwein-Pondorf-Verley method" or a modification thereof, suitably using an alkanol as a reaction component and as solvent, as isopropanol, and using a metalalkanolate, as metalisopropanolate, e.g. aluminium isopropanolate.

Further, (i) the oxo group in a compound corresponding to these of formula I and which carries an oxo group at a carbon atom bound to a nitrogen atom may be reduced by two hydrogen atoms.

Said compounds are e.g. such of the formula XX

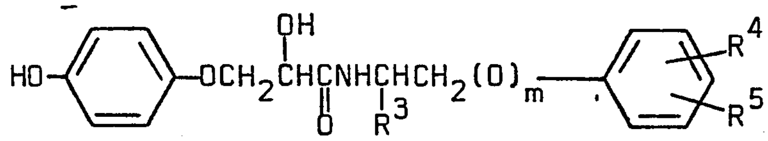

XX

wherein $R^3$, $R^4$, $R^5$, and m have the same meanings as given above, or of the formula XXI

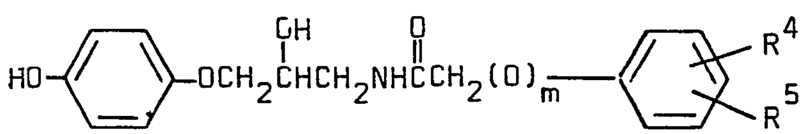

XXI

wherein $R^4$, $R^5$, and m have the meanings given above.

Further, an oxo group, or a derivative of a hydroxy group or halogen in a benzylic position can be reduced to methylene e.g. by means of catalytical hydrogenation (Pd/C) or by Wolff-Kischner reduction or the like. Thus, the compounds of the formulae XXII and XXIII can be reduced.

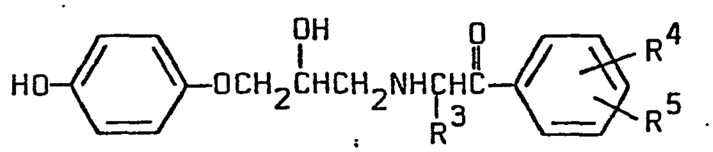

XXII

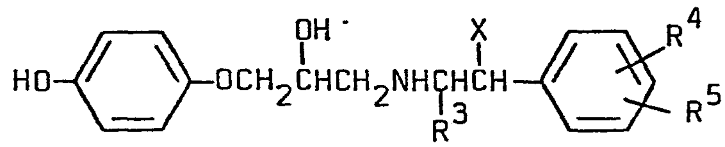

XXIII

wherein $R^3$, $R^4$, and $R^5$ have the meanings given above and X is -OH or halogen.

The reduction can be carried out according to the above described manner using complex metalhydrides, e.g. lithiumaluminiumhydride or di-isobutylaluminiumhydride. Suitably the reaction takes place in an inert solvent as an ether, e.g. diethylether or tetrahydrofuran. Complex borohydrides can also be used especially when a selective reaction is wanted.

Further, (k) a compound of the present invention can be obtained by eliminating one or more substituents in one or both of the aromatic rings. Such substituents are F, Br, Cl, and I which can be eliminated by means of hydrogenolysis, or amino groups which are removable by diazotizing and hydrolysis. The splitting off of a substituent can also take place in connection with prior mentioned methods.

In a compound of formula I a phenol hydroxy group in either or both the aromatic rings can be formed by cleavage of a corresponding ether, e.g. a benzyl, allyl, silyl, alkyl e.g. methyl ether, a tetrahydropyranyl or other ether obtainable by ether formation with a vinyl ether. The ether cleavage constitutes an ancillary step to the synthetic methods mentioned above in such cases when the starting materials contain such an ether group in the place of a phenol hydroxy group or such a group is introduced during the synthesis.

Depending on the process conditions and the starting material the end product is obtained either in free form or in the form of its acid additions salt, which is included in the scope of the invention. Thus, for example, basic neutral or mixed salts may be obtained as well as hemiamino-, sesqui- or polyhydrates. The acid addition salts of the new compounds may in a manner known per se be transformed into free compounds using e.g. basic agents as alkali or ion exchanger. On the other ohand, the free bases obtained may form salts with organic or inorganic acids. In the preparation of acid addition salts preferably such acids are used which form suitable therapeutically acceptable salts. Such acids are e.g. hydrohalogen acids, sulphuric acid, phosphoric acid, nitric acid, perchloric acid, aliphatic, alicyclic, aromatic or heterocyclic carboxy or sulphonic acids, such as formic, acetic, propionic, succinic, glycolic, lactic, malic, tartaric, citric, ascorbic, maleic, hydroxymaleic or pyruvic acid, phenylacetic, benzoic, p-aminobenzoic, anthranilic, p-hydroxybenzoic, salicyclic or p-aminosalicyclic acid, pamoic acid, methanesulphonic, ethanesulphonic, hydroxyethanesulphonic, ethylenesulphonic acids, halogenbenzenesulphonic, toluenesulphonic, naphthylsulphonic acids, or sulphanilic acid; methionine, tryptohpane, lysine or arginine.

These or other salts of the new compounds as e.g. picrates may serve as purifying agents or the free bases obtained as the free bases are transformed into salts, these are separated and the bases are then set free from the salts again. According to the close relationship between the new compounds in free form and in the form of their salts it will be understood from the above and the below that, if possible, the corresponding salts are included in the free compound.

The invention also relates to any embodiment of the process of which one starts from any compound obtained as an intermediate in any process step and one carries out the lacking process step, or one breaks off the process at any step, or at which one forms a starting material under the reaction conditions, or at which a reaction component possibly in the form of its salt is present.

Thus, one may react an aldehyde of the formula XXIV

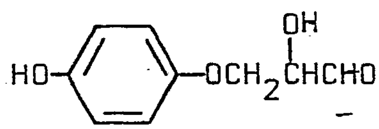

XXIV

with an amine of the formula XXV

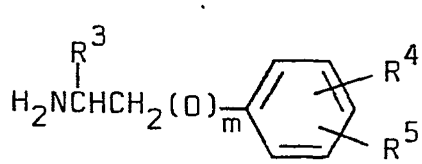

XXV

wherein $R^3$, $R^4$, $R^5$, and m have the meanings given above, in the presence of a suitable reducing agent, as one of the above mentioned. Thereby a compound of formula XIV is obtained as an intermediate, which then is reduced according to the invention.

Further, one may in a manner known per se react an amine of the formula IV with a keton of the formula XXVI .

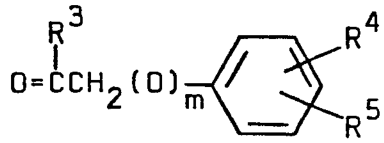

XXVI

wherein $R^3$, $R^4$, $R^5$, and m have the meanings given above, in the presence of a suitable reducing agent, as one of the above mentioned to produce compounds of formula XV or XVI as an intermediate, which then is reduced according to the invention.

The new compounds may, depending on the choice of starting materials and process, be present as optical antipodes or racemate, or if they contain two asymmetric carbon atoms be present as an isomer mixture (racemate mixture).

The isomer mixtures (racemate mixtures) obtained may depending on physical-chemical differences of the component, be separated into the both stereoisomeric (diastereomeric) pure racemate e.g. by means of chromatography and/or fractionated crystallization.

The racemates obtained can be separated according to known methods, e.g. by means of recrystallization from an optical active solvent, by means of microorganisms, or by a reaction with optically active acids forming salts of the compound and separating the salts thus obtained, e.g. by means of their different solubility in the diastereomers, from which the antipodes by the influence of a suitable agent may be set free.

Suitable useable optically active acids are e.g. the L- and D-forms of tartaric acid, di-o-tolyltartaric acid, malic acid, mandelic acid, camphersulphonic acid or quinic acid. Preferably the more active part of the two antipodes is isolated. Further the two enantiomers can be obtained by asymmetrical reduction of the corresponding keto-compound.

Suitably such starting materials are used for carrying out the reactions of the invention, which material leads to groups of end products primarily especially desired and especially to the specifically described and preferred end products.

The starting materials are known or may, if they should be new, be obtained according to processes known per se.

In clinical use the compounds of the invention are administered normally orally, rectally or by injection in the form of a pharmaceutical preparation, which contains an active component either as free base or as pharmaceutically acceptable, non-toxic acid addition salts, e.g. the hydrochloride lactate, acetate, sulphamate or the like in combination with a pharmaceutical carrier.

Thereby the mentioning of the new compounds of the invention is here related to either the free amine base or the acid addition salts of the free base, even if the compounds are generally or specifically described, provided that the context in which such expressions are used, e.g. in the examples, with this broad meaning should not correspond. The carrier may be a solid, semisolid or liquid diluent or a capsule. These pharmaceutical preparations are a further object of the invention. Usually the amount of active compound is between 0.1 to 99 % by weight of the preparation, suitably between 0.5 to 20 % by weight in preparations for injection and between 2 to 50 % by weight in preparations for oral administration.

In the preparation of pharmaceutical preparations containing a compound of the present invention in the form of dosage units for oral administration the compound elected may be mixed with a solid, pulverulent carrier, as e.g. with lactose, saccharose, sorbitol, mannitol, starch, as potatoe starch, corn starch, amylopectin, cellulose derivatives or gelatine, as well as with an antifriction agent as magnesium stearate, calcium stearate, polyethyleneglycol waxes, or the like, and be pressed into tablets. If coated tablets are wanted, the above prepared core may be coated with concentrated solution of sugar, which solution may contain e.g. gum arabicum, gelatine, talc, titandioxide or the like. Furthermore, the tablets may be coated with a lacquer dissolved in an easily volatile organic solvent or mixture of solvents. To this coating a dye may be added in order to easily distinguish between tablets with different active compounds or with different amounts of the active compound present.

In the preparation of soft gelatine capsules (pearl-shaped, closed capsules), which consist of gelatine and e.g. glycerine or in the preparation of similar closed capsules the active compound is mixed with a vegetable oil. Hard gelatine capsules may contain granules of the active compound in combination with a solid, pulverulent carrier as lactose, saccharose, sorbitol, mannitol, starch (as e.g. potatoe starch, corn starch or amylopectin), cellulose derivatives or gelatine.

Dosage units for rectal administration may be prepared in the form of suppositories, which contain the active substance in a mixture with a neutral fat base, or they may be prepared in the form of gelatine-rectal capsules which contain the active substance in a mixture with a vegetable oil or paraffin oil.

Liquid preparations for oral administration may be present in the form of sirups or suspensions, e.g. solutions containing from about 0.2 % by weight to about 20 % by weight of the active substance described, whereby the residue consists of sugar and a mixture of ethanol, water, glycerol, and propylene glycol. If desired, such liquid preparations may contain colouring agents, flavouring agents, saccharine and carboxymethylcellulose as a thickening agent.

Solutions for parenteral administration by injection may be prepared as an aqueous solution of a watersoluble pharmaceutically acceptable salt of the active compound, preferably in a concentration from about 0.5 % by weight to about 10 % by weight. These solutions may also contain stabilizing agents and/or buffering agents and may suitably be available in different dosage unit ampoules.

The preparation of pharmaceutical tablets for peroral use is carried out in accordance with the following method.

The solid substances included are ground or sieved to a certain particle size. The binding agent is homogenized and suspended in a certain amount of solvent. The therapeutic compound and necessary auxiliary agents are mixed during a continuous and constantly mixing with the binding agent solution and are moistened so that the solution is uniformly divided in the mass without overmoistening any parts. The amount of solvent is usually so adapted that the mass obtains a consistency reminding of wet snow. The moistening of the pulverulent mixture with the binding agent solution causes the particles to gather together slightly to aggregates and the real granulating process is carried out in such a way that the mass is pressed through a sieve in the form of a net of stainless steel having a mesh size of about 1 mm. The mass is then placed in thin layers on a tray to be dried in a drying cabinet. This drying takes place during 10 hours

and has to be standardized carefully as the damp degree of the granulate is of utmost importance for the following process and for the feature of the tablets. Drying in a fluid bed may possibly be used. In this case the mass is not put on a tray but is poured into a container having a net bottom.

After the drying step the granules are sieved so that the particle size wanted is obtained. Under certain circumstances powder has to be removed.

To the so called final mixture, disintegrating, antifriction agents and antiadhesive agents are added. After this mixture the mass shall have its right composition for the tabletting step.

The cleaned tablet punching machine is provided with a certain set of punches and dies, whereupon the suitable adjustment for the weight of the tablets and the degree of compression is tested out. The weight of the tablet is decisive for the size of the dose in each tablet and is calculated starting from the amount of therapeutic agent in the granules. The degree of compression affects the size of the tablet, its strength and its ability to disintegrate in water. Especially as regards the two latter properties the choice of compression pressure (0.5 to 5 ton) means something of a balance step. When the right adjustment is set, the preparation of tablets is started which is carried out with a rate of 20,000 to 200,000 tablets per hour. The pressing of the tablets requires different times and depends on the size of the batch.

The tablets are freed from adhering pulver in a specific apparatus and are then stored in closed packages until they are delivered.

Many tablets, especially these which are rough or bitter, are coated with a coating. This means that these are coated with a layer of sugar or some other suitable coating.

The tablets are usually packed by machines having an electronic counting device. The different types of packages consist of glass or plastic gallipots but also boxes, tubes and specific dosage adapted packages.

The daily dose of the active substance varies and is depending on the type of administration, but as a general rule it is 100 to 400 mg/day of active substance at peroral administration and 5 to 20 mg/day at intravenous administration.

BEST MODE OF CARRYING OUT THE INVENTION

The following illustrates the principle and the adaption of the invention, however, without being limited thereto. Temperature is given in degrees Celsius.

Example 1

19.4 g of 2-(2,6-dimethoxyphenyl)-1-methylethylamine were dissolved in 100 ml of isopropanol and 12.8 g of 3-(4--benzyloxyphenoxy)-1,2-epoxypropane in 300 ml of isopropanol were added in 2 h during reflux. The mixture was boiled for 10 h and evaporated. The residue was treated with 200 ml of 2 M HCl and washed twice with ether. The aqueous phase was extracted with methylene chloride. The organic phase was recrystallized from acetonitril. 6.4 g of this compound were dissolved in 120 ml of ethanol and hydrogenated over 10% Pd/C at NTP. After 340 ml of hydrogen had been taken up, the solution was filtered and evaporated. Yield 4.0 g. The hydrochloride of 1-[2-(2,6-dimethoxyphenyl)-1-methylethylamino]-3-(4-hydroxyphenoxy)propanol-2 melted at

150-152$^{o}$C. The structure was determined using NMR and equivalent weight.

Example 2

3-(4-hydroxyphenoxy)-1-[2-(4-hydroxyphenoxy)-1-methylethylamino]propanol-2 was prepared in accordance with Example 1 using 5.5 g of 2-(4-hydroxyphenoxy)-1-methylethylamine and 7.7 g of 1-(4-benzyloxyphenoxy)-2,3-epoxypropane as starting materials. Yield 2.4 g. Melting point 190$^{o}$C (HCl). The structure was determined using NMR and equivalent weight.

Example 3

1-[2-(2,6-dichlorophenyl)-1-methylethylamino]-3-(4-hydroxyphenoxy)propanol-2 was prepared in accordance with Example 1 using 6.1 g of 2-(2,6-dichlorophenyl)-1-methylethylamine and 6.1 g of 1-(4-benzyloxy)-2,3-epoxypropane as starting materials. Yield 0.8 g. Melting point 240$^{o}$C (HCl). The structure was determined using NMR.

Example 4 (method g)

2.7 g of 3-amino-1-(4-benzyloxyphenoxy)-2-propanol and 2.6 g of 3-benzyloxyphenoxy-2-propanone was refluxed for 1 h in 50 ml of methanol. 0.38 g of $NaBH_4$ was added and the solution refluxed for 1 h and left overnight. The solvent was evaporated and the residue acidified with HCl (aq) to pH 2. The aqueous layer was extracted with $CHCl_3$. The solvent was evaporated and the residue dissolved in 100 ml of ethanol. After addition of the catalyst (5% Pd/C) the mixture was hydrogenated (440 ml $H_2$). The mixture was filtered, evaporated, and the residue was crystallized by treating with ether. Yield 1.2 g of 3-(4-hydroxyphenoxy)-1--[2-(3-hydroxyphenoxy)-1-methylethylamino]propanol-2. Melting point 115$^{o}$C (HCl). The structure was determined using NMR analysis.

Example 5

3-(4-hydroxyphenoxy)-1-[2-(3-methoxyphenoxy)-1-methylethylamino]propanol-2 was prepared in accordance with Example 4 using 9.0 g of 3-methoxyphenoxy-2-propanone and 13.9 g of 3-amino-1-(4-benzyloxyphenoxy)-2-propanol as starting materials. Yield 3.5 g. Melting point 148$^{o}$C (HCl). The structure was determined using NMR and equivalent weight.

Example 6

1-[2-(3-chlorophenoxy)-1-methylethylamino]-3-(4-hydroxyphenoxy)propanol-2 was prepared in accordance with Example 4 using 9.2 g of 3-chlorophenoxy-2-propanone and 13.2 g of 3-amino-1-(4-benzyloxyphenoxy)-2-propanol as starting materials. The benzyl group was, however, removed by refluxing in 100 ml of 99% ethanol and 60 ml of conc. HCl (aq) for 2 h. Yield 3.9 g. Melting point 158$^{o}$C (HCl). The structure was determined using NMR and equivalent weight.

Example 7

1-[2-(3-hydroxyphenoxy)ethylamino]-3-(4-hydroxyphenoxy)-propanol-2 was prepared in accordance with Example 1 using 19.3 g of 2-(3-benzyloxyphenoxy)ethylamine and 8 g of 1-(4-benzyloxyphenoxy)-2,3-epoxypropane as starting materials. Yield 1.8 g. Melting point 160-163$^{o}$C (HCl). The structure was determined using NMR-analysis.

Example 8

1-(2-phenoxy-1-methylethylamino)-3-(4-hydroxyphenoxy)-propanol-2 was prepared in accordance with Example 1 using 6.0 g of 1-(4-benzyloxyphenoxy)-2,3-epoxypropane and 7.0 g of 2-phenoxy-1-methylethylamine as starting materials. Yield 4.5 g. Melting point 155$^{o}$C (HCl). The structure was determined using NMR-analysis.

Example 11

1-[2-(2-chlorophenoxy)ethylamino]-3-(4-hydroxyphenoxy)-propanol-2 was prepared in accordance with Example 10 using 6.0 g of N-benzyl-2-(2-chlorophenoxy)ethylamine and 6.4 g of 1-(4-benzyloxyphenoxy)-2,3-epoxypropane as starting materials. Yield 2.7 g. Melting point 154°C (HCl). The structure was determined using NMR and equivalent weight.

Example 12

3-(4-hydroxyphenoxy)-1-[2-(2-methoxyphenoxy)ethylamino]-propanol-2

21.9 g of 3-(4-benzyloxyphenoxy)-1,2-epoxypropane and 20.0 g of N-benzyl-2-[2-methoxyphenoxy]ethylamine were refluxed in 400 ml of isopropanol for 12 h. The mixture was evaporated, crystallized from ether, and recrystallized from methanol. Yield 13.7 g. This product was hydrogenated in 0.75 l of ethanol over 10% Pd/C. 1.4 l of $H_2$ was consumed. The mixture was filtered and evaporated. The residue was dissolved in ethyl acetate. 0.5 g of a by-product crystallized and was filtered off. The compound was crystallized with acetic acid. Yield 4.5 g. Melting point 130-132°C ($CH_3COOH \times H_2O$). The structure was determined using NMR-analysis.

Example 13 (method b)

2.7 g of 3-amino-1-(4-benzyloxyphenoxy)-2-propanol, 2.4 g of 2-bromo-1-(3-methoxyphenoxy)propane and 1.4 g of $K_2CO_3$ were refluxed in 100 ml of isopropanol for 15 h. After filtering the solution was evaporated. The residue was dissolved in ethanol, treated with hydrogen chloride, and hydrogenated over Pd/C until the reaction stopped. After filtering and evaporating the hydrochloride of 3-(4-hydroxyphenoxy)-1-[2-(3-methoxyphenoxy)-1-methylethylamino]-propanol-2 crystallized. Melting point 148°C.

Example 14 (method c)

2.4 g of Na were dissolved in 100 ml of ethanol, whereupon 20.0 g of 4-benzyloxyphenol and 27.5 g of 3-chloro-1-[2-(3-methoxyphenoxy)-1-methylethylamino]propanol-2 were added. The mixture was heated in an autoclave on a boiling waterbath for 10 hours. Thereupon it was filtered and the filtrate was evaporated to dryness. The residue was dissolved in 500 ml of ethanol and 10 ml of HCl (aq). The mixture was hydrogenated over 5% Pd/C until 2.4 l of hydrogen had been taken up. The solution was thereafter filtered and evaporated to dryness. The residue was recrystallized from acetonitrile. The hydrochloride of 3-(4-hydroxyphenoxy)-1-[2-(3-methoxyphenoxy)-1-methylethylamino]-propanol-2 melted at 148°C.

In a similar manner the end compound was prepared from 3-methoxyphenol and 3-(4-benzyloxyphenoxy)-1-(2-chloro-1-methylethylamino)propanol (method e).

Example 15 (method d)

0.12 moles of 4-benzyloxyphenol were mixed with 0.080 moles of 1-[2-(3-methoxyphenoxy)-1-methylethyl]-3-acetidinol, 0.500 moles of benzylalcohol and 0.002 moles of KOH. The mixture was refluxed while stirring for 6 hours at 140°C and was then cooled and extracted with 2M HCl. The extract was diluted with 300 ml of ethanol and hydrogenated over 5% Pd/C. After filtering and evaporation of ethanol the residue was recrystallized from acetonitrile. The hydrochloride of 3-(4-hydroxyphenoxy)-1-[2-(3-methoxyphenoxy)-1-methylethylamino]propanol-2 melted at 148°C.

Example 16 (method h)

1.0 g of 3-(4-hydroxyphenoxy)-1-[2-(3-methoxyphenoxy)-1-methylethylamino]propanone-2 was dissolved in 25 ml of

methanol and the solution was cooled to 0°C on an ice-bath. 0.25 g of $NaBH_4$ were added little by little while stirring first at 0°C for 1 hour and then at ambient temperature for 0.5 hour. The solution thus obtained was evaporated whereupon 50 ml of $H_2O$ were added. The aqueous phase was extracted 3 times with ethyl acetate. The collected ethyl acetate phase was dried over $MgSO_4$ and filtered. The hydrochloride was precipitated by introducing an equivalent amount of hydrochloric acid. The hydrochloride of 3-(4-hydroxyphenoxy)-1-[2-(3-methoxyphenoxy)-1-methylethyl-amino]propanol-2 melted at 148°C.

Example 17 (method f)

5.3 g of 3-(4-benzyloxyphenoxy)-1-[N-benzyl-2-(3-methoxy-phenoxy)-1-methylethylamino]propanol-2 were dissolved in 400 ml of ethanol. 10 ml of hydrochloric acid were added. 0.2 g of Pd/C (10%) catalyst was added and the mixture was hydrogenated until 460 ml of hydrogen had been absorbed. The catalyst was filtered off and the solvent was evaporated. The residue was recrystallized from acetonitrile. The hydrochloride of 3-(4-hydroxyphenoxy)-1-[2-(3-methoxyphenoxy)-1-methylethylamino]propanol-2 melted at 148°C.

Example 18 (method k)

1-[2-(2-bromophenoxy)ethylamino]-3-(4-benzyloxyphenoxy)-propanol-2 (4.7 g) was dissolved in 150 ml of ethanol and 5 ml of conc. HCl was added. Thereafter 5% Pd/C was added as a catalyst and the mixture was hydrogenated at NTP until the uptake of $H_2$-gas stopped (490 ml). The catalyst was filtered off and the mixture was evaporated to dryness. The residue was crystallized from acetonitrile/methanol 4/1. Yield of 1-(2-phenoxyethylamino)-3-(4-hydroxyphenoxy)-propanol-2 = 2.1 g. Melting point 163-164°C (HCl). The structure was determined using NMR-analysis.

Example 19 (method i)

6.3 g of N-[3-(4-hydroxyphenoxy)-2-hydroxypropyl]phenoxy-acetamide dissolved in 100 ml of THF was slowly added to a stirred slurry of 1.5 g $LiAlH_4$ in THF ($N_2$-atmosphere). After the addition was complete (1/2 h), the mixture was refluxed for 2h. Thereafter the mixture was treated with water and acidified with hydrochloric acid. The mixture was extracted with ether and the aqueous phase was neutralized to pH 9.5 and extracted with ethyl acetate. The ethyl acetate phase was dried over $Na_2SO_4$, filtered and evaporated. The residue was dissolved in acetonitrile/methanol 4/1 and treated with HCl (g). The hydrochloride of 1-(2-phenoxy-ethylamino)-3-(4-hydroxyphenoxy)propanol-2 crystallized. Melting point 163-164$^{\circ}$C. The structure was determined using NMR analysis.

Example 20

3-(4-hydroxyphenoxy)-1-[2-(3-methoxyphenoxy)-1-methylethyl-amino]propanol-2 hydrochloride (1 g), sodium chloride (0.8 g) and ascorbic acid (0.1 g) were dissolved in sufficient amount of distilled water to give 100 ml of solution. This solution, which contains 10 mg of active substance per each ml, was used in filling ampoules, which were sterilized.

Example 21

A sirup containing 2% (weight per volume) of active substance was prepared from the following ingredients:

| | | |
|---|---|---|
| 1-[2-(3-chlorophenoxy)-1-methylethylamino]-3--(4-hydroxyphenoxy)propanol-2 · HCl | | 2.0 g |
| Saccharine | | 0.6 g |
| Sugar | | 30.0 g |
| Glycerine | | 5.0 g |
| Flavouring agent | | 0.1 g |
| Ethanol 96% | | 10.0 ml |
| Distilled water | ad | 100.0 ml |

Sugar, saccharine and the ether salt were dissolved in 60 g of warm water. After cooling glycerine and a solution of flavouring agents dissolved in ethanol were added. To the mixture water was then added to 100 ml.

The above given active substance may be replaced with other pharmaceutically acceptable acid addition salts.

Example 22

1-[2-(3-chlorophenoxy)-1-methylethylamino]-3-(4-hydroxyphenoxy)propanol-2 hydrochloride (250 g) was mixed with lactose (175.8 g), potatoe starch (169.7 g) and colloidal silicic acid (32 g). The mixture was moistened with 10% solution of gelatine and was granulated through a 12-mesh sieve. After drying potatoe starch (160 g), talc (50 g) and magnesium stearate (5 g) were admixed and the mixture thus obtained was pressed into tablets (10,000) which contain 25 mg of substance. The tablets are sold on the market provided with a breaking score to give another dose than 25 mg or to give multiples thereof when broken.

Example 23

Granules were prepared from 3-(4-hydroxyphenoxy)-1-[2--(3-methoxyphenoxy)-1-methylethylamino]propanol-2 • hydrochloride (250 g), lactose (175.9 g) and an alcoholic solution of polyvinylpyrrolidone (25 g). After the drying step the granules were mixed with talc (25 g), potatoe starch (40 g) and magnesium stearate (2.50 g) and was pressed into 10.000 tablets being biconvex. These tablets are primarily coated with a 10% alcoholic solution of shellac and thereupon with an aqueous solution containing saccharose (45%), gum arabicum (5%), gelatine (4%) and dyestuff (0.2%). Talc and powder sugar were used for powdering after the first five coatings. The coating was then coated with a 66% sugar sirup and polished with a 10% carnauba wax solution in carbon tetrachloride.

BIOLOGICAL EFFECTS

Positive inotropic effects of the compounds according to the invention

The positive inotropic effect and the cardioselectivity of the compounds were studied in cats with an average weight of 3 kg. The animals were pretreated with reserpine (5 mg/kg i.p.) about 16 hours before experiment. This treatment eliminated the reflex sympathetic control of the heart and the vascular tonus. Following pentobarbital anaesthesia (Mebumal®, 30 mg/kg i.p.) artificial air respiration was started. The two vagus nerves were cut and the adrenal glands were excluded from the circulation by means of ligatures on the adrenal veins.

Blood pH, $pCO_2$ and $pO_2$ were kept within the normal ranges throughout the experiments. All recordings were made on a Grass Polygraph. Heart rate, pressure in the left ventricle (LVP), the first derivative of LVP (dP/dt), intra-arterial

blood pressure and peripheral resistance were recorded. LVP was recorded via a catheter tip pressure transducer (Millar), which was inserted via the left carotid artery. This pressure curve was differentiated and max dP/dt was recorded by means of a peak detector. The recording of max dP/dt was linear up to 20 000 mg Hg/s. The increase in max dP/dt was used as a measure of the increased contractility of the heart.

To study the effect on peripheral vascular resistance the blood passing through a femoral artery was fed via a catheter and a pump back to the distal part of the vessel. With a pressure transducer the perfusion pressure was registered. At a perfusion with a constant flow the perfusion pressure is directly proportional to the peripheral resistance of the vascular bed of the hind limb.

The substances were administered intravenously in increasing doses. Dose-response curves were constructed for max dP/dt and the vasodilatation by plotting the per cent of the maximal response against the logarithm of the dose of the drug.

The compounds according to the invention caused a dose dependent increase in contractility with half maximal effects at doses 0.01-0.36 μmol/kg. Isoprenaline has a very short duration of action which requires continuous infusion. Half maximal effect of isoprenaline was obtained at 0.004 μmol/kg·min. The maximal effects on cardiac contractility amounted to 70-96 per cent of maximal response to isoprenaline. The compounds had no effects on peripheral blood vessels, indicating that the compounds are $\beta_1$-selective agonists.

The duration of action of the compounds has been studied in conscious beagle dogs. An electromagnetic flow probe was inplanted on the ascending aorta for determination of the

inotropic effect measured as maximal flow acceleration (max dF/dt) in the aorta. For determination of blood pressure catheters were inplanted in thoracic aorta and left atrium. Increasing doses of the substances were administered intravenously and the decline of inotropic effect (max dF/dt) was used for determination of duration.

The time for 50% decline of the positive inotropic effect (max dF/dt) varied from 60 up to 180 min for the compounds according to the invention, comparative value for isoprenaline was less than 1 min. Thus all the compounds showed a long duration of action as compared to isoprenaline.

CLAIMS

1. A compound of the formula I

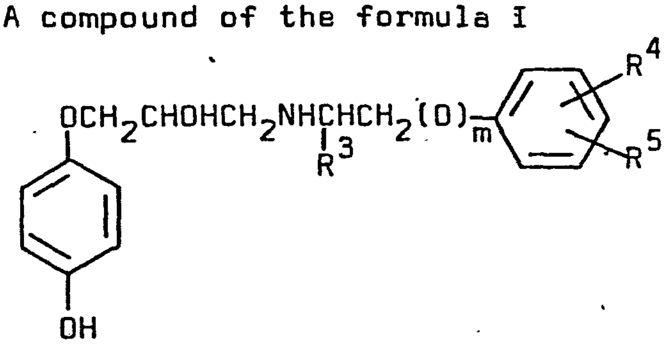

(I)

wherein $R^3$ is selected from the group consisting of hydrogen or methyl, m is an integer 0 or 1, whereby when m is 0, $R^4$ and $R^5$ are the same and selected from the group consisting of methoxy, fluoro, bromo, and chloro in 2- and 6-position, whereby when m is 1, $R^4$ is hydrogen and $R^5$ is selected from the group consisting of hydrogen, methoxy, ethoxy, acetyl, methyl, hydroxymethyl, cyano, chloro, fluoro, bromo, hydroxy, or a pharmaceutically acceptable salt thereof.

2. A compound according to claim 1 wherein m is 0 and $R^4$ and $R^5$ are the same and selected from methoxy, fluoro, bromo and chloro in the 2- and 6-position.

3. A compound according to claim 1 wherein m is 1, $R^4$ is H and $R^5$ is selected from hydrogen, methoxy, chloro, fluoro, bromo and hydroxy.

4. A compound according to claim 3 having the formula

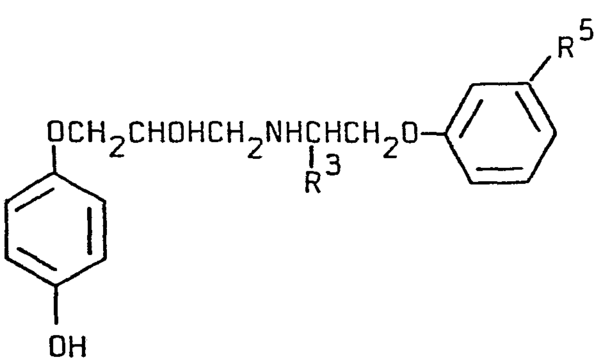

wherein $R^3$ is hydrogen or methyl and $R^5$ is selected from hydrogen, fluoro, chloro, bromo and hydroxy.

5. The compound according to claim 1, which is

1-[2-(2,6-dimethoxyphenyl)-1-methylethylamino]-3-(4-hydroxyphenoxy)propanol-2,

1-[2-(2,6-dichlorophenyl)-1-methylethylamino]-3-(4-hydroxyphenoxy)propanol-2,

3-(4-hydroxyphenoxy)-1-[2-(3-hydroxyphenoxy)-1-methylethylamino]propanol-2,

1-[2-(3-chlorophenoxy)-1-methylethylamino]-3-(4-hydroxyphenoxy)propanol-2,

1-[2-(3-hydroxyphenoxy)ethylamino]-3-(4-hydroxyphenoxy)propanol-2,

1-(2-phenoxy-1-methylethylamino)-3-(4-hydroxyphenoxy)propanol-2, or

1-(2-phenoxyethylamino)-3-(4-hydroxyphenoxy)propanol-2

or a pharmaceutically acceptable salt thereof.

6. A process for the preparation of a new amine compound of formula I

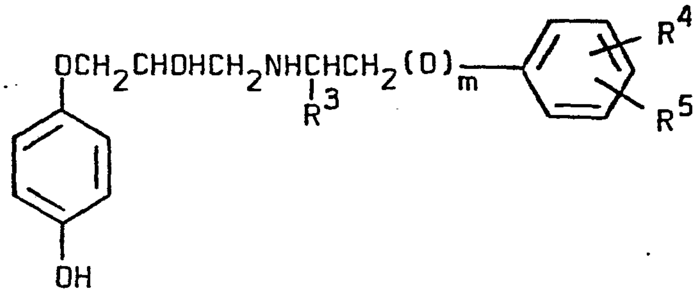

(I)

wherein $R^3$ is selected from the group consisting of hydrogen or methyl, m is an integer 0 or 1, whereby when m is 0, $R^4$ and $R^5$ are the same and are each selected from the group consisting of methoxy, fluoro, bromo, and chloro in 2- and 6-position, whereby when m is 1, $R^4$ is hydrogen and $R^5$ is selected from the group consisting of hydrogen, methoxy, ethoxy, acetyl, methyl, hydroxymethyl, cyano, chloro, fluoro, bromo, hydroxy, or a pharmaceutically acceptable salt thereof, characterized in that

a) a compound of the formula II

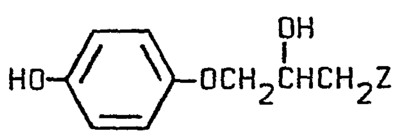

(II)

wherein Z is a hydroxy group or a reactive, esterified hydroxy group, or OH and Z together form an epoxy group, is reacted with an amine of the formula III

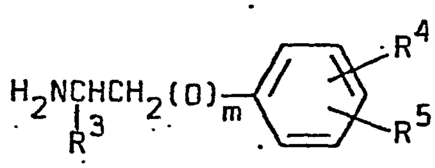

(III)

wherein $R^3$, $R^4$, $R^5$, and m have the meanings given above;

b) a compound of the formula IV

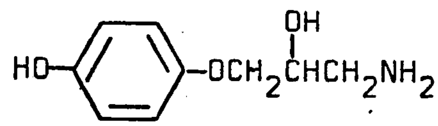

(IV)

wherein $R^1$ and $R^2$ have the same meanings as given above, is reacted with a compound of the formula V

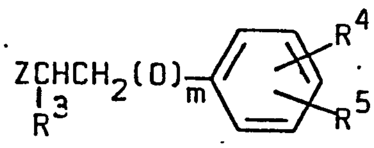

(V)

wherein $R^3$, $R^4$, $R^5$, m, and Z have the same meanings as given above; or with a compound of formula VI

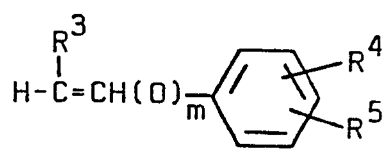

(VI)

wherein $R^3$, $R^4$, $R^5$, and m have the meanings given above, or with a compound of the formula VII

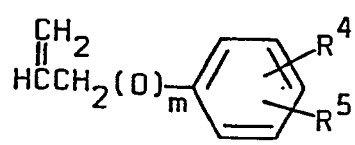

(VII)

wherein $R^4$, $R^5$, and m have the meanings given above,

c) a compound of the formula VIII

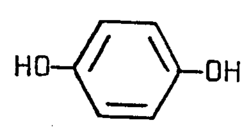

(VIII)

is reacted with a compound of formula IX

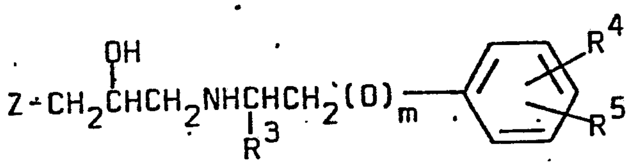

(IX) :

wherein $R^3$, $R^4$, $R^5$, m, and Z have the same meanings as given above; or

d) a compound of the formula VIII

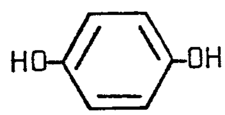

(VIII)

is reacted with a compound of the formula XII

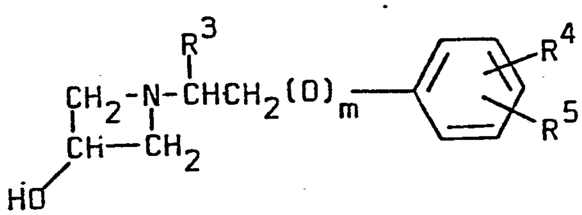

(XII)

wherein $R^3$, $R^4$, $R^5$, and m have the same meanings as given above;

e) a compound of the formula X

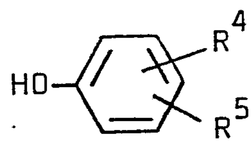

(X)

wherein $R^4$ and $R^5$ have the same meanings as given above, is reacted with a compound of the formula XI

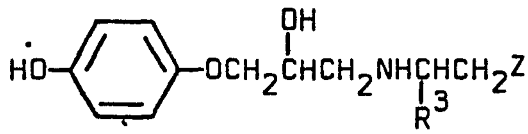

(XI)

wherein $R^3$ and Z have the same meanings as given above;

f) from a compound of the formula I wherein $R^3$, $R^4$, $R^5$, and m have the same meanings as above, and which compound has a splitable residue at the nitrogen atom of the amino group and/or has a splitable residue at the hydroxy groups, this residue is split off; or

g) a Schiff's base of the formula XIV or XV

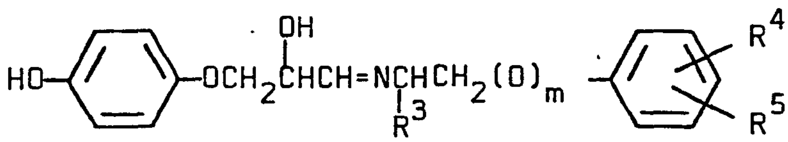

(XIV)

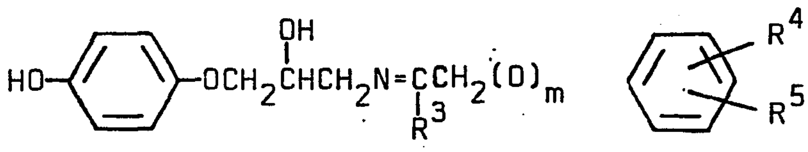

(XV)

or a cyclic tautomer of formula XVI corresponding to the compound of formula XV

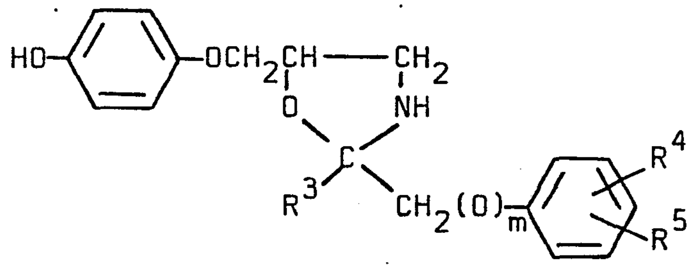

(XVI)

or a compound corresponding to formula I and having an unsaturation α to the amino nitrogen, wherein $R^3$, $R^4$, $R^5$, and m have the same meanings as above, whereby the compounds XV and XVI may be present simultaneously, is reduced, or

h) in a compound of the formula XIX

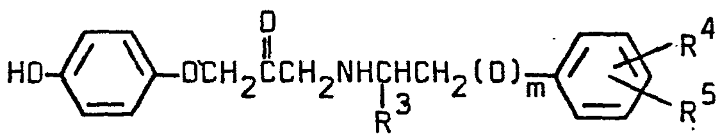

(XIX)

wherein $R^3$, $R^4$, $R^5$, and m have the same meanings as above, the oxo group is reduced to a hydroxy group.

i) reducing a compound of formula XX

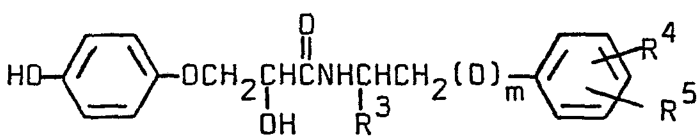

(XX)

wherein $R^3$, $R^4$, $R^5$, and m have the same meanings as above; or

a compound of the formula XXI

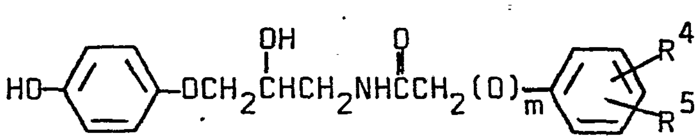

(XXI)

wherein $R^4$, $R^5$, and m have the same meanings as given above, or

a compound of the formula XXII

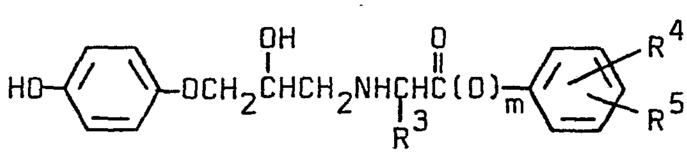

(XXII)

wherein $R^3$, $R^4$, $R^5$, and m have the same meanings as given above, or

a compound of formula XXIII

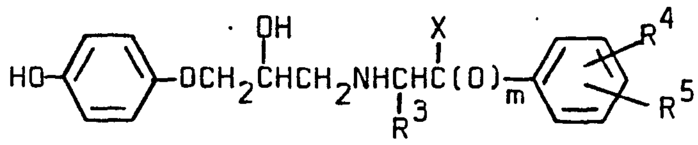

(XXIII)

wherein $R^3$, $R^4$, $R^5$, and m have the same meanings as given above, and X is hydroxy or halogen; or

k) hydrogenating a compound of the formula

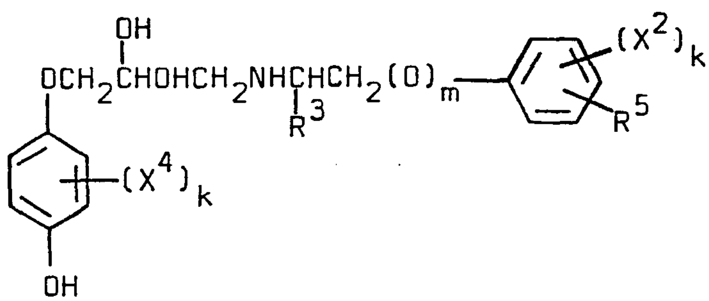

wherein $R^3$, $R^4$, $R^5$, and m have the meanings given above, $X^2$ denotes $R^4$ or a residue that can be split off, and $X^4$ denotes a residue that can be split off, and k is an integer 1 to 4, to split off the splitable residue; and if desired, substituents are introduced, split off, or reacted in the compounds of the definition of the end product, or compounds obtained are transformed into other end products and/or isomer mixtures obtained are separated into pure isomers, and/or racemates obtained are separated into optical antipodes and/or free bases obtained are transformed into their therapeutically acceptable salt or salts obtained are transformed into their free bases.

7. A pharmaceutical preparation intended for the treatment of cardiac failure in a mammal, which preparation comprises at least one β-receptor stimulating phenoxyhydroxypropylamine compound according to claim 1, in assocication with a

pharmaceutically acceptable carrier, said compound being present in an amount effective to provide β-receptor stimulating activity in said mammal.

8. A pharmaceutical preparation according to claim 7, wherein the β-receptor stimulating compound of claim 1 is present in association with a liquid pharmaceutically acceptable carrier.

9. A pharmaceutical preparation according to claim 7, wherein the β-receptor stimulating compound of claim 1 is present in association with a solid pharmaceutically acceptable carrier.

European Patent Office

# EUROPEAN SEARCH REPORT

0064487

Application number

EP 82 85 0093

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | JOURNAL OF PHARMACEUTICAL SCIENCES, vol. 68, no. 10, October 1979, pages 1236-1238, Washington D.C., USA J.P. O'DONNELL et al.: "Synthesis and pharmacology of potential beta-blockers" * Whole document: compound V * | 1,2,6-9 | C 07 C 93/06<br>A 61 K 37/13 |
| X | EP-A-0 006 614 (MERCK & CO.) * Claims; page 14, example 7 * | 1,3-9 | |
| A | CHEMICAL ABSTRACTS, vol. 72, no. 25, 22nd June 1970, page 323, no. 132292t, Columbus, Ohio, USA & CS - A - 131 156 (M. WENKE et al.) 15-02-1969 * Abstract * | 1,6-9 | |
| | | | TECHNICAL FIELDS SEARCHED (Int. Cl. 3) |
| | | | C 07 C 93/00<br>A 61 K 31/00 |

The present search report has been drawn up for all claims

| Place of search | Date of completion of the search | Examiner |
|---|---|---|
| THE HAGUE | 03-08-1982 | PAUWELS G.R.A. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503. 03.82